(19) Europäisches Patentamt
European Patent Office
Office européen des brevets

(11) Publication number: **0 063 481**
**B1**

(12) **EUROPEAN PATENT SPECIFICATION**

(45) Date of publication of patent specification: **17.10.84**

(51) Int. Cl.³: **C 07 H 17/02,** A 61 K 31/70

(21) Application number: **82301957.5**

(22) Date of filing: **16.04.82**

(54) **Water-soluble antitumor, antiviral and immunosuppressant compounds.**

(30) Priority: **20.04.81 JP 59930/81**
**12.02.82 JP 21632/82**

(43) Date of publication of application:
**27.10.82 Bulletin 82/43**

(45) Publication of the grant of the patent:
**17.10.84 Bulletin 84/42**

(84) Designated Contracting States:
**DE FR GB IT SE**

(56) References cited:
**DE-A-2 326 916**
**DE-A-2 740 281**

**JOURNAL OF THE CHEMICAL SOCIETY, CHEMICAL COMMUNICATIONS, no.3, 1981, J.P. FERRIS et al.: "General synthesis of imidazole C-nucleosides from carbohydrate adducts of diaminomaleonitrile", pages 110-112**

(73) Proprietor: **SUMITOMO CHEMICAL COMPANY, LIMITED**
**15 Kitahama 5-chome Higashi-ku**
**Osaka-shi Osaka-fu (JP)**

(72) Inventor: ***Toshio, Atsumi***
**3-45 Seiwadai Nishi 2-chome**
**Kawanishi (JP)**
Inventor: **Yuzo, Tarumi**
**1-402 Ryodocho 4**
**Nishinomiya (JP)**
Inventor: **Tetsutaro, Sanjiki**
**7-6, Funakicho**
**Ibaraki (JP)**
Inventor: **Yoshiaki, Takebayashi**
**11-8-409, Sone Higashimachi 2-chome**
**Toyonaka (JP)**
Inventor: **Noboru, Yoshida**
**5-3-618, Higashiawaji 1-chome**
**Higashiyodogawa-ku Osaka (JP)**

(74) Representative: **Geering, Keith Edwin et al**
**REDDIE & GROSE 16 Theobalds Road**
**London WC1X 8PL (GB)**

**EP 0 063 481 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to 4-carbamoylimidazolium-5-olate derivatives useful as antitumor agents, antiviral agents and immunosuppressants, their preparation and pharmaceutical compositions containing them.

It is known that the compound of the following formula (II) has antitumor and immunosuppresive activity (Canadian Patent No. 1,078,736):

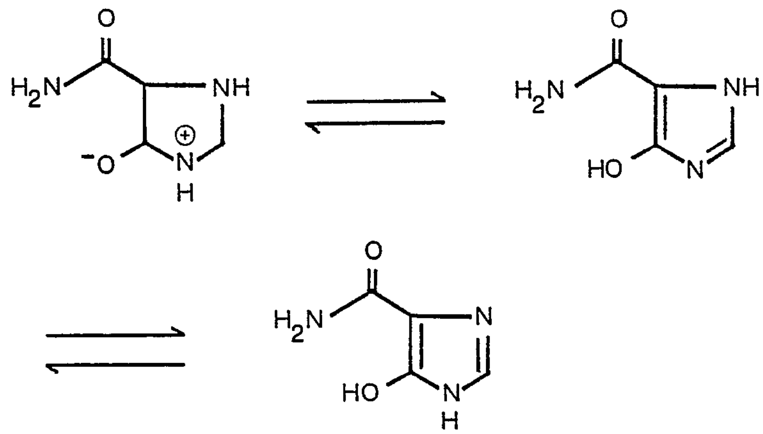

(II)

The compound of the formula (II) has insufficient solubility in aqueous media, and for use in therapy more soluble compounds are desired.

As a result of extensive study seeking new active derivatives which have higher solubility in aqueous media, the present invention provides compounds of the following formulae (I—a), (I—b):

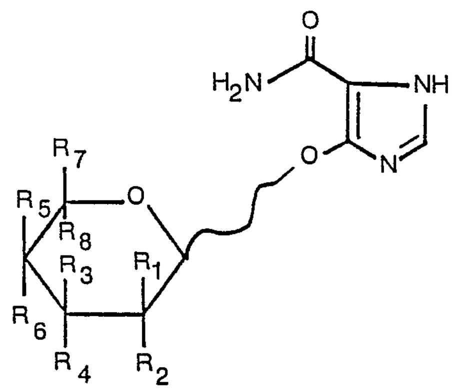

(I—a)

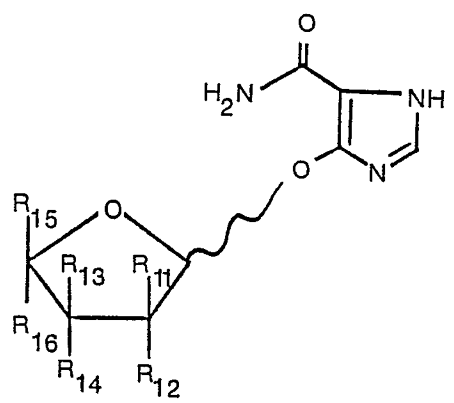

(I—b)

wherein either $R_1$ or $R_2$ is a hydrogen atom, a hydroxy group, an acyloxy group, a phthalimido group or an acetamido group and the other is a hydrogen atom; either $R_3$ or $R_4$ is a hydroxy group or an acyloxy group and the other is a hydrogen atom; either $R_5$ or $R_6$ is a hydroxy group or an acyloxy group and the other is a hydrogen atom; either $R_7$ or $R_8$ is a hydrogen atom, a methyl group, a hydroxymethyl group, an acyloxymethyl group, an alkoxycarbonyl group, a group of the formula:

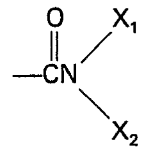

wherein $X_1$ and $X_2$ are the same or different and selected from a hydrogen atom and $C_1$ to $C_6$ alkyl groups, or a carboxyl group and the other is a hydrogen atom; either $R_{11}$ or $R_{12}$ is a hydrogen atom, a hydroxy group or an acyloxy group and the other is a hydrogen atom; either $R_{13}$ or $R_{14}$ is a hydroxy group or an acyloxy group and the other is a hydrogen atom; and either $R_{15}$ or $R_{16}$ is a methyl group, a hydroxymethyl group or an acyloxymethyl group and the other is a hydrogen atom.

As used herein, the term "acyloxy" covers e.g. alkanoyloxy having 2 to 7 carbon atoms (e.g. acetyloxy, propionyloxy, butyryloxy, isobutyryloxy, valeryloxy, isovaleryloxy, pivaloyloxy, hexanoyloxy, heptanoyloxy) and benzoyloxy (which may be substituted, e.g. with alkyl of 1 to 4 carbon atoms, alkoxy of 1 to 4 carbon atoms, nitro or halogen, as in o-, m- or p-toluoyloxy, methoxybenzoyloxy, nitrobenzoyloxy); the term "acyloxymethyl" means methyl substituted with such an acyloxy group; the term "alkoxycarbonyl" means carbonyl substituted with alkoxy of e.g. 1 to 4 carbon atoms (e.g. methoxy, ethoxy, propoxy); the term "lower alkyl" means alkyl having 1 to 6 carbon atoms (e.g. methyl, ethyl, propyl). The term "halogen" includes fluorine, chlorine, bromine and iodine, chlorine and bromine being preferred.

A compound of formula (I—a) or (I—b) can be prepared by reacting a 4-carbamoylimidazolium-5-olate derivative of the formula (III):

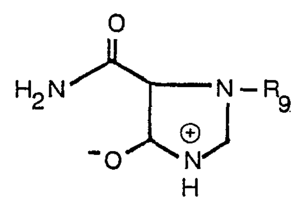

(III)

wherein $R_9$ is a hydrogen atom or a substituted or unsubstituted benzyl group; or a reactive derivative thereof with a carbohydrate derivative of the formula (IV—a) or (IV—b):

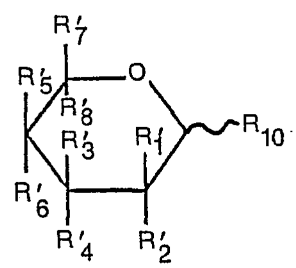

(IV–a),

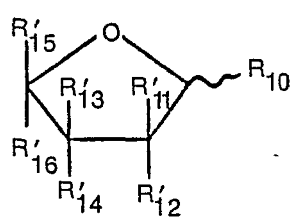

(IV–b)

(wherein either $R'_1$ or $R'_2$ is a hydrogen atom, an acyloxy group, a phthalimido group of an acetamido group and the other is a hydrogen atom; either $R'_3$ or $R'_4$ is an acyloxy group and the other is a hydrogen atom; either $R'_5$ or $R'_6$ is an acyloxy group and the other is a hydrogen atom; either $R'_7$ or $R'_8$ is a hydrogen atom, a methyl group, an acyloxymethyl group, an alkoxycarbonyl group or a group of the formula:

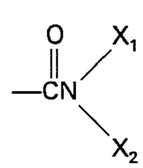

wherein $X_1$ and $X_2$ are as defined above, and the other is a hydrogen atom; either $R'_{11}$ or $R'_{12}$ is a hydrogen atom or an acyloxy group and the other is a hydrogen atom; either $R'_{13}$ or $R'_{14}$ is an acyloxy group and the other is a hydrogen atom; either $R'_{15}$ or $R'_{16}$ is a methyl group or an acyloxy-methyl group and the other is a hydrogen atom; and $R_{10}$ is a halogen atom, a lower (i.e. $C_1$ to $C_4$) alkoxy group or a lower (i.e. $C_2$ to $C_7$) alkanoyloxy group in the presence or absence of Lewis acid to obtain a compound of the formula (V—a) or (V—b):

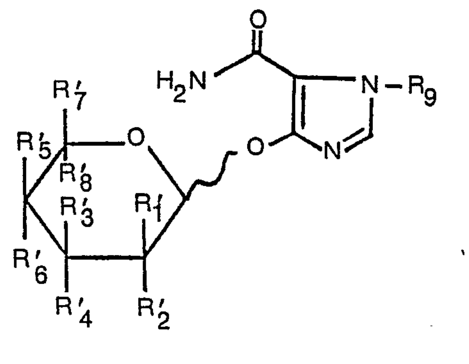

(V—a)

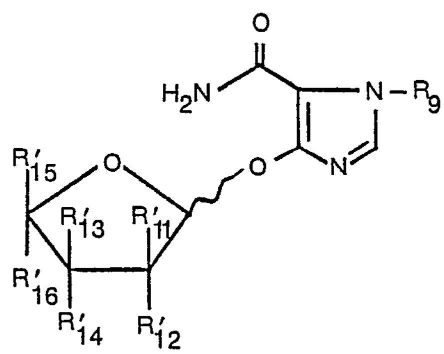

(V—b)

wherein $R'_1$, $R'_2$, $R'_3$, $R'_4$, $R'_5$, $R'_6$, $R'_7$, $R'_8$, $R'_{11}$, $R'_{12}$, $R'_{13}$, $R'_{14}$, $R'_{15}$, $R'_{16}$, and $R_9$ are as defined above.

Examples of preferred reactive derivatives of the compound of formula (III) are trialkyltin derivatives. The trialkyltin derivatives can be prepared, for example, by refluxing the compound of formula (III) with bis(tributyltin)oxide in benzene for a few hours and evaporating the solvent under reduced pressure.

Typical examples of said Lewis acids are silver perchlorate, stannic chloride, zinc chloride, zinc bromide, titanium tetrachloride, silver trifluoromethanesulfonate and trimethylsilylperchlorate.

The term "substituted benzyl" covers benzyl substituted with e.g. one or two nitro groups, alkyl having one to four carbon atoms or alkoxy having one to four carbon atoms.

Examples of the carbohydrate derivatives of the formula (IV—a, b) are those of D-glucose, L-glucose, D-galactose, D-mannose, L-mannose, D-glucosamine, D-galactosamine, D-mannosamine, L-rhamnose, D-fucose, L-fucose, D-glucuronic acid, 2-deoxy-D-glucose, 2-deoxy-D-galactose, D-ribofuranose, D-ribopyranose, D-xylofuranose, D-xylopyranose, D-arabinofuranose, D-arabinopyranose, D-lyxofuanose, D-lyxopyranose, L-xylofuranose, L-xylopyranose, L-arabinofuranose, L-arabinopyranose, L-lyxofuranose, L-luxopyranose, 2-deoxy-D-ribofuranose, 5-deoxy-D-ribofuranose, and 5-deoxy-L-arabinose.

Typical examples of preferred solvents which may be used in the reaction between the compound of formula (III) and the compound of the formula (IV—a or —b) are ethers (e.g. tetrahydrofuran, dioxane, dimethoxyethane), polar solvents (e.g. acetonitrile, N,N-dimethylformamide, dimethylsulfoxide), hydrocarbon solvents (e.g. benzene, toluene, xylene), and halogenated hydrocarbon solvents (e.g. carbon tetrachloride, dichloromethane, chloroform, 1,2-dichloroethane).

The reaction can generally be effected by controlling the reaction temperature at from —70 to 50°C., preferably from —20 to 25°C.

The compounds of formula (V—a, or —b) can be isolated and purified by known purification methods (e.g. recrystallization, column chromatography).

When $R_9$ is a substituted or unsubstituted benzyl group, being a protective group of the compounds of formula (V—a or —b) it can be removed by catalytic reduction in the presence or absence of acid to obtain the compound of formula (I'—a or —b):

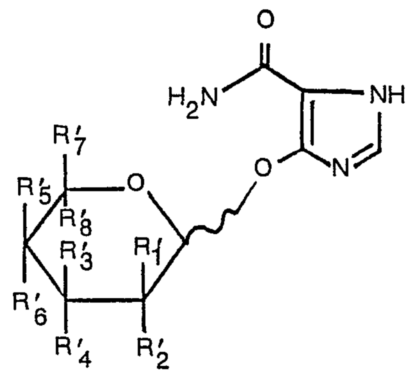

(I'–a),

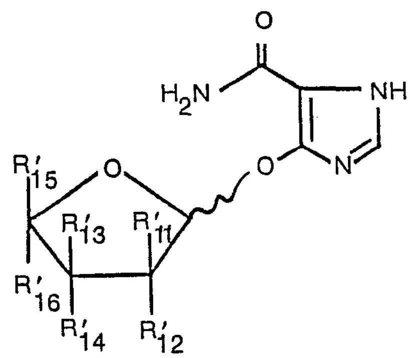

(I'–b)

wherein $R'_1$, $R'_2$, $R'_3$, $R'_4$, $R'_5$, $R'_6$, $R'_7$, $R'_8$, $R'_{11}$, $R'_{12}$, $R'_{13}$, $R'_{14}$, $R'_{15}$, and $R'_{16}$ are as defined above.

In this reaction, a preferred benzyl group is a nitrobenzyl group and the reaction can be accelerated, so as to finish in a few hours under atmospheric pressure, by addition of 1 to 1.5 mol equivalents of acid. Typical examples of said acids are acetic acid, propionic acid, methanesulfonic acid, p-toluenesulfonic acid, hydrochloric acid, nitric acid and phosphoric acid. Typical examples of the catalyst which may be used in this reaction are palladium-carbon, palladium black, platinum oxide and Raney nickel.

Typical examples of the solvents which may be used in this reaction are ethers (e.g. tetrahydrofuran, dioxane), alcohols (e.g. methanol, ethanol) and water, preferably methanol containing the water.

Protective groups in the sugar moieties of compounds of formula (I'—a or —b) can be removed by treating (I'—a or —b) with methanol saturated with ammonia or metal alkoxide (e.g. sodium methoxide, sodium ethoxide) in alcohols (e.g. methanol, ethanol) to obtain the compounds of formula (I''—a or —b):

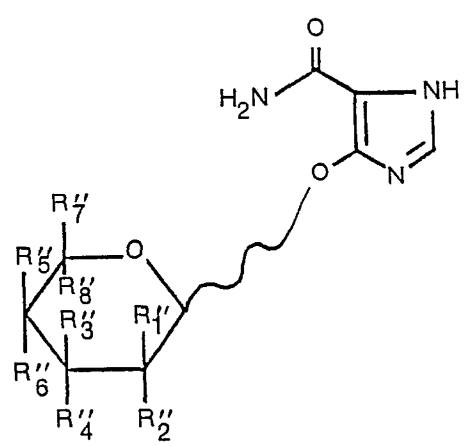

(I''–a),

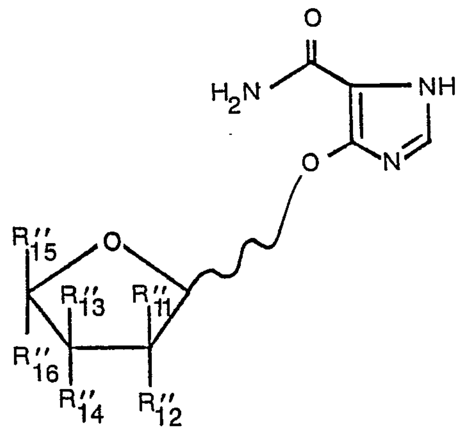

(I''—b)

wherein either $R_1''$ or $R_2''$ is a hydrogen atom, a hydroxy group, a phthalimido group or an acetamido group and the other is a hydrogen atom; either $R_3''$ or $R_4''$ is a hydroxy group and the other is a hydrogen atom; either $R_5''$ or $R_6''$ is a hydroxy group and the other is a hydrogen atom; either $R_7''$ or $R_8''$ is a hydrogen atom, a methyl group, a hydroxymethyl group, an-alkoxycarbonyl group or a group of the formula:

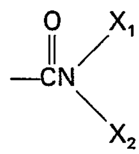

(wherein $X_1$ and $X_2$ are as previously defined) and the other is a hydrogen atom; either $R''_{11}$ or $R''_{12}$ is a hydrogen atom or a hydroxy group and the other is a hydrogen atom; either $R''_{13}$ or $R''_{14}$ is a hydroxy group and the other is a hydrogen atom; and either $R''_{15}$ or $R''_{16}$ is a methyl group or a hydroxymethyl group and the other is a hydrogen atom.

A compound of formula (I''—a) wherein either $R_7''$ or $R_8''$ is an alkoxycarbonyl group may be hydrolyzed to give a carboxyl derivative which may form a salt with a base (e.g. ammonia, triethylamine, sodium) or may produce the corresponding amide derivative by reaction with an organic amine (e.g. ammonia, methylamine, diethylamine).

The compounds of formula (I—a and —b) may each exist in a mixture of two tautomers as follows:

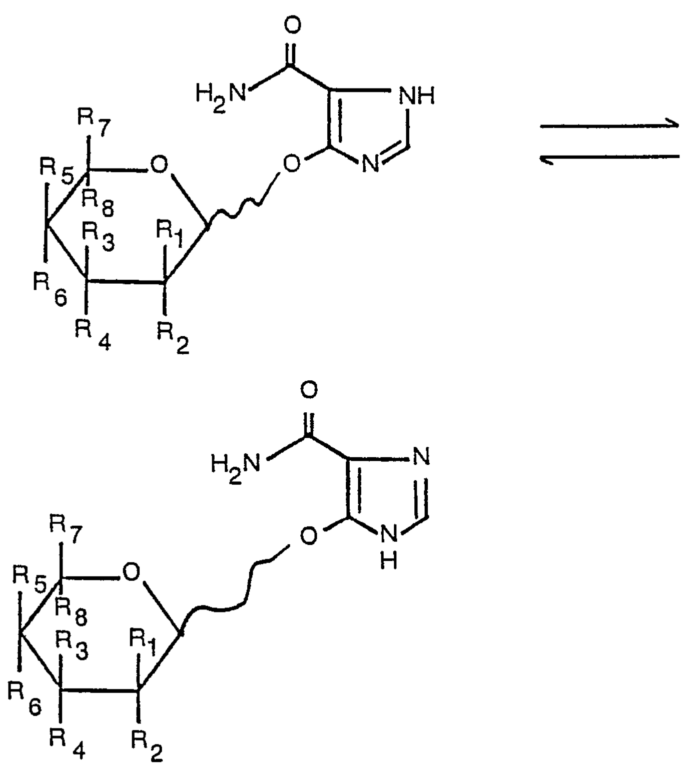

(I—a)

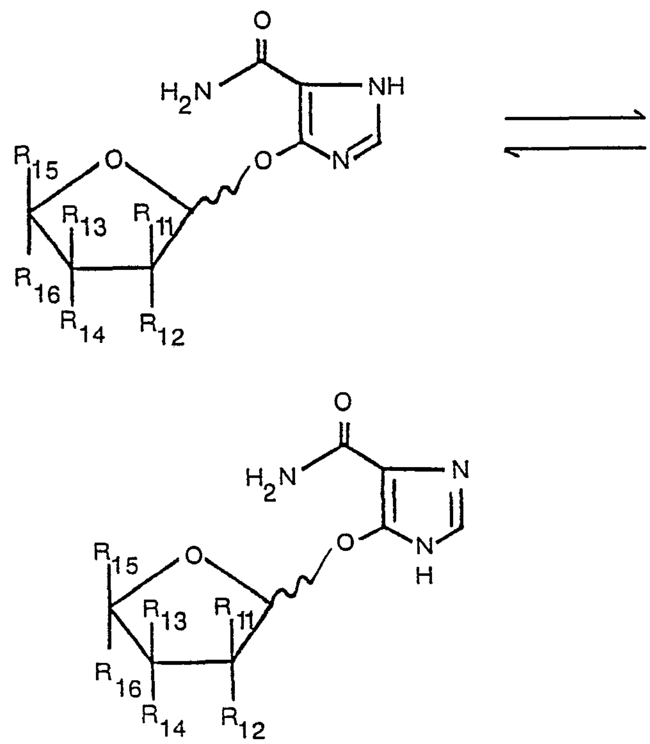

(I—b)

wherein $R_1$, $R_2$, $R_3$, $R_4$, $R_5$, $R_6$, $R_7$, $R_8$, $R_{11}$, $R_{12}$, $R_{13}$, $R_{14}$, $R_{15}$, and $R_{16}$ are as defined above; these tautomers are within the scope of the present invention.

A compound of formula (III) where $R_9$ is a substituted or unsubstituted benzyl group can be prepared by the following method:

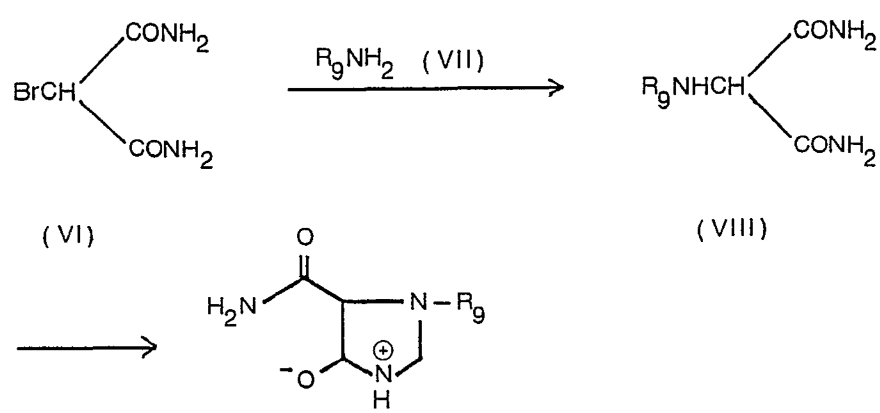


(VI) (VIII)

Compounds of the present invention have shown potent antitumor activities against Sarcoma 180, Lewis Lung Carcinoma, Ehrlich Carcinoma, and P—388 leukemia.

The compounds of formula (I—a, b) are useful as antitumor agents and they exhibit tumor inhibiting effects and life span prolongation effects.

The antitumor activities of compounds of the present invention were estimated according to the methods described in "Cancer Chemotherapy Reports" Part 3, Vol. 3 (No. 2) p. 13 (1972).

The results are given in the following Table 1:

TABLE 1

Antitumor effect on experimental mouse tumors

| Compound | (Dose) (mg/kg) | Route | Schedule | Inhibition ratio (%) Lewis Lung Carcinoma (solid) |
|---|---|---|---|---|
| 4-(2,3,4,6-tetra-O-acetyl-β-D-glucopyranosyl)oxy-1H-imidazole-5-carboxamide | 360 | po | 9qd | 85 |
| | 180 | po | 9qd | 76 |
| | 90 | po | 9qd | 53 |
| 4-β-D-glucopyranosyloxy-1H-imidazole-5-carboxamide | 300 | ip | 9qd | 58 |
| | 200 | ip | 9qd | 45 |
| 4-(2,3,4,6-tetra-O-acetyl-β-D-galactopyranosyl)oxy-1H-imidazole-5-carboxamide | 360 | po | 9qd | 89 |
| | 180 | po | 9qd | 84 |
| | 90 | po | 9qd | 58 |
| Methyl 1-O-(5-carbamoyl-1H-imidazole-4-yl)-2,3,4-tri-O-acetyl-β-D-glucopyranuronate | 175 | po | 9qd | 55 |
| | 87 | po | 9qd | 53 |
| Methyl 1-O-(5-carbamoyl-1H-imidazole-4-yl)-β-D-glucopyranuronate | 200 | po | 9qd | 44 |
| 4-(2,3,5-tri-O-acetyl-β-D-ribofuranosyl)oxy-1H-imidazole-5-carboxamide | 50 | ip | 9qd | 50 |
| | 100 | po | 9qd | 60 |
| 4-β-D-ribofuranosyloxy-1H-imidazole-5-carboxamide | 50 | ip | 9qd | 58.6 |
| 4-(2,3,4,tri-O-acetyl-α-L-rhamnopyranosyl)-oxy-1H-imidazole-5-carboxamide | 314 | po | 9qd | 66 |
| | 157 | po | 9qd | 43 |

$BDF_1$ male mice, 5 weeks old, weighing between 18 and 22 grams were used. Each test group was composed of 6 to 7 mice. Two million cells of Lewis Lung Carcinoma were injected in the hind leg. The drug was administered intraperitoneally (ip) or orally (po) each day from 1 to 9 (i.e. 9qd). After killing the mice on day 13, tumors were removed and weighed. The tumor inhibitory ratio was calculated according to the following formula.

$$\text{Inhibition ratio} = \left(1 - \frac{\text{the mean tumor weights of treated group}}{\text{the mean tumor weights of control group}}\right) \times 100$$

The compounds of the present invention possess immunosuppressive and antiviral activity as well as antitumor activity.

The compounds (I—a, b) of the present invention have low toxicity. They did not show toxic symptoms, even when over 500 mg/kg does were orally administered to mice. Moreover, they do not exhibit an influence on decrease of peripheral leucocytes, which is one of the most serious side effects of immunosuppressants.

The compounds of the present invention can be administered orally or parenterally to a warm-blood animal at a daily dose of 2—200 mg/kg as an antitumor agent, and 1—100 mg/kg as an immunosuppressant agent in a conventional dosage unit form.

The compounds of the present invention are made up alone or together with a conventional pharmaceutical carrier or diluent into a conventional solid or liquid pharmaceutical preparation (e.g. powders, granules, tablets, capsules, suspensions, emulsions, solutions) using methods conventional in the pharmaceutical field. For example, tablets or capsules contain 50 to 500 mg of compounds (I—a, b). Notably the compounds (I—a, b) of the present invention can be used for an injection and a drop, due to their water solubility.

The following Examples are shown to illustrate the present invention more precisely but it is not intended to limit the present invention thereto.

### Reference Example 1

To a solution of 41.60 g of p-nitrobenzylamine in 660 ml of dry ethanol was added 49.34 g of $\alpha$-bromomalonamide and 38.2 ml of triethylamine. After stirring for two hours under reflux, the reaction mixture was cooled to room temperature. Separated crystals were filtered off, washed with ethanol and isopropyl ether, and dried to give 53.36 g of $\alpha$-(4-nitrobenzyl)aminomalonamide. A mixture of 50.45 g of this $\alpha$-(4-nitrobenzyl)aminomalonamide, 177.8 g of triethyl orthoformate and 0.76 g of p-toluenesulfonic acid monohydrate in 1.3 liters of dry ethanol was stirred for three hours under reflux in an argon atmosphere. The reaction mixture was cooled on an ice-bath and separated crystals were filtered off, washed with ethanol and isopropyl ether, and dried to give 50.78 g of 5-carbamoyl-1-(4-nitrobenzyl)imidazolium-4-olate.

The crude material was recrystallized from 50% methanol-water, m.p. 266°C. (dec.).

$\nu_{max}^{nujol}$ (cm$^{-1}$): 3300, 3170, 3120, 1650, 1595, 1570, 1515

| Elemental analysis: | C (%) | H (%) | N (%) |
|---|---|---|---|
| Calculated for $C_{11}H_{10}N_4O_4 \cdot \frac{1}{2}H_2O$ | 48.71 | 4.09 | 20.66 |
| Found | 48.91 | 4.03 | 20.77 |

### Example 1

To a solution of 2.570 g of silver trifluoromethanesulfonate, dried under reduced pressure, in 40 ml of dry nitromethane was added 1.35 ml of tetramethylurea. A solution of 4.112 g of 2,3,4,6-tetra-O-acetyl-$\alpha$-D-glucopyranosyl bromide in 15 ml of dry nitromethane was added to the reaction mixture at a temperature from 0°C. to 15°C. After stirring for 15 minutes, the reaction mixture was cooled on an ice-salt bath (at a temperature of −11°C.) and 25 ml of benzene solution of the tributyltin derivative of 1.311 g of 5-carbamoyl-1-(4-nitrobenzyl)-imidazolium-4-olate (which was prepared by refluxing a mixture of the 5-carbamoyl-1-(4-nitrobenzyl)imidazolium-4-olate and bis-(tri-n-butyltin)-oxide in dry benzene), was added. After stirring for an hour, the reaction mixture was gradually heated up to room temperature and the stirring was continued for 17 hours. The separated crystals were filtered off, and washed with dry benzene. The filtrate was concentrated under reduced pressure to give 11.43 g of oily residue, which was purified by silica gel column chromatography (eluting with a 5:1 v/v mixture of methylene chloride and acetone to give 2.744 g of 1-(4-nitrobenzyl)-4-(2,3,4,6-tetra-O-acetyl-$\beta$-D-glucopyranosyl)oxy-1H-imidazole-5-carboxamide.

The crude material was recrystallized from ethanolisopropyl ether—n-hexane. m.p. 100—102°C.

| Elemental analysis: | C (%) | H (%) | N (%) |
|---|---|---|---|
| Calculated for $C_{25}H_{28}N_4O_{13} \cdot \frac{1}{3}H_2O$ | 49.92 | 4.86 | 9.32 |
| Found | 50.12 | 4.81 | 9.23 |

$\nu_{max}^{nujol}$ (cm$^{-1}$): 3460, 3350, 3170, 3110, 1750, 1660, 1600

Example 2

To a solution of 0.150 g of 1-(4-nitrobenzyl)-4-(2,3,4,6-tetra-O-acetyl-β-D-glucopyranosyl)oxy-1H-imidazole-5-carboxamde in 10 ml of tetrahydrofuran were added 0.3 ml of N—HCl aqueous solution and 80 mg of 10% palladium-carbon and catalytic reduction was continued for an hour. To the reaction mixture was added 250 mg of sodium bicarbonate, followed by stirring for half an hour.

Separated crystals were filtered off and the filtrate was concentrated under reduced pressure. Chromatographical purification (on silica gel with a 15:1 v/v chloroform-methanol mixture of the residue gave 92 mg of 4-(2,3,4,6-tetra-O-acetyl-β-D-glucopyranosyl)oxy-1H-imidazole-5-carboxamide, which was recrystallized from ethanol—isopropyl ether to give colorless needle crystals. m.p. 237—238°C.

| Elemental analysis: | C (%) | H (%) | N (%) |
|---|---|---|---|
| Calculated for $C_{18}H_{23}N_3O_{11} \cdot H_2O$ | 45.47 | 5.30 | 8.84 |
| Found | 45.75 | 5.04 | 8.48 |

$\nu_{max}^{nujol}$ ($cm^{-1}$): 3490, 3340, 3140, 1750, 1665, 1650, 1605

| | |
|---|---|
| λmax in water | 248 nm (ε 13500) |
| λmax in N—HCl aqueous solution | 231 nm (ε 9000) |
| λmax in N—NaOH aqueous solution | 264 nm (ε 14500) |

Example 3

To a solution of 457 mg of 4-(2,3,4,6-tetra-O-acetyl-β-D-glucopyranosyl)oxy-1H-imidazole-5-carboxamide in 30 ml of dry methanol was added 270 mg of 95% sodium methoxide. The reaction mixture was stirred for 20 minutes at room temperature and 0.33 ml of acetic acid was added. Chromatographical purification (on LH—20 (product of Pharmacia A/S) with methanol) of the residue which was obtained after evaporation of the reaction mixture under reduced pressure, gave 260 mg of 4-β-D-glucopyranosyloxy-1H-imidazole-5-carboxamide which was crystallized with acetone. m.p. 167°C. (dec.).

| Elemental analysis: | C (%) | H (%) | N (%) |
|---|---|---|---|
| Calculated for $C_{10}H_{15}N_3O_7 \cdot \frac{1}{2}H_2O$ | 40.27 | 5.41 | 14.09 |
| Found | 40.38 | 5.43 | 13.97 |

$\nu_{max}^{KBr}$ ($cm^{-1}$): 3430, 3320, 3200, 2910, 1675, 1610

| | |
|---|---|
| λmax in water | 248 nm (ε 11800) |
| λmax in N—HCl aqueous solution | 241 nm (ε 8400) |
| λmax in N—NaOH aqueous solution | 265 nm (ε 12500) |

Example 4

Following a procedure similar to that of Example 1 but using 2.622 g of 5-carbamoyl-1-(4-nitrobenzyl)imidazolium-4-olate and 8.220 g of 2,3,4,6-tetra-O-acetyl-α-D-galactopyranosyl bromide there was obtained 3.857 g of 1-(4-nitrobenzyl)-4-(2,3,4,6-tetra-O-acetyl-β-D-galactopyranosyl)oxy-1H-imidazole-5-carboxamide.

Recrystallization from ethanol—isopropyl ether—n-hexane. m.p. 111°C (dec.)

$\nu_{max}^{nujol}$ ($cm^{-1}$): 3450, 3350, 1755, 1655, 1600, 1350, 1245, 1225, 1080, 1025, 960, 735

| Elemental analysis: | C (%) | H (%) | N (%) |
|---|---|---|---|
| Calculated for $C_{25}H_{28}N_4O_{13} \cdot 0.5H_2O$ | 49.92 | 4.86 | 9.32 |
| Found | 49.85 | 4.90 | 9.02 |

Example 5

Following a procedure similar to that of Example 2 but using 2.664 g of 1-(4-nitrobenzyl)-4-(2,3,4,6-tetra-O-acetyl-β-D-galactopyranosyl)oxy-1H-imidazole-5-carboxamide there was obtained 1.90 g of 4-(2,3,4,6-tetra-O-acetyl-β-D-galactopyranosyl)oxy-1H-imidazole-5-carboxamide. m.p. 234.5—235°C.

$\nu_{max}^{nujol}$ ($cm^{-1}$): 3460, 3150, 1750, 1660, 1610, 1505, 1430, 1215, 1070, 1035, 915, 690

| Elemental analysis: | C (%) | H (%) | N (%) |
|---|---|---|---|
| Calculated for $C_{18}H_{23}N_3O_{11}\cdot\frac{1}{4}H_2O$ | 46.80 | 5.13 | 9.10 |
| Found | 46.89 | 5.10 | 9.15 |

Example 6

Following a procedure similar to that of Example 3 but using 1.143 g of 4-(2,3,4,6-tetra-O-acetyl-β-D-galactopyranosyl)oxy-1H-imidazole-5-carboxamide there was obtained 0.570 g of 4-β-D-galactopyranosyloxy-1H-imidazole-5-carboxamide. m.p. 130°C.

$\nu_{max}^{KBr}$ ($cm^{-1}$): 3350, 2920, 1655, 1605, 1500, 1430, 1330, 1115, 1065, 1020

| Elemental analysis: | C (%) | H (%) | N (%) |
|---|---|---|---|
| Calculated for $C_{10}H_{15}N_3O_7\cdot\frac{1}{2}MeOH\cdot\frac{1}{2}H_2O$ | 40.13 | 5.77 | 13.37 |
| Found | 39.90 | 5.68 | 13.33 |

Example 7

Following a procedure similar to that of Example 1 but using 1.311 g of 5-carbamoyl-1-(4-nitrobenzyl)imidazolium-4-olate and 3.658 g of 2-acetamido-3,4,6-tri-O-acetyl-2-deoxy-α-D-glucopyranosyl chloride there was obtained 1.0 g of 1-(4-nitrobenzyl)-4-(2-acetamido-3,4,6-tri-O-acetyl-2-deoxy-β-D-glucopyranosyl)oxy-1H-imidazole-5-carboxamide.

Recrystallization from methanol—isopropyl ether—n-hexane. m.p. 114—117°C.

$\nu_{max}^{KBr}$ ($cm^{-1}$): 3450, 1750, 1650, 1600, 1520, 1470, 1370, 1345, 1230, 1050

| Elemental analysis: | C (%) | H (%) | N (%) |
|---|---|---|---|
| Calculated for $C_{25}H_{29}N_5O_{12}\cdot 1.2H_2O$ | 48.97 | 5.16 | 11.42 |
| Found | 49.29 | 5.01 | 11.05 |

Example 8

Following a procedure similar to that of Example 2 but using 0.861 g of 1-(4-nitrobenzyl)-4-(2-acetamido-3,4,6-tri-O-acetyl-2-deoxy-β-D-glucopyranosyl)-oxy-1H-imidazole-5-carboxamide there was obtained 0.523 g of 4-(2-acetamido-3,4,6-tri-O-acetyl-2-deoxy-β-D-glucopyranosyl)oxy-1H-imidazole-5-carboxamide.

Recrystallization from chloroform—ether. m.p. 133—135°C.

$\nu_{max}^{KBr}$ ($cm^{-1}$): 3450, 1750, 1650, 1600, 1240, 1040

| Elemental analysis: | C (%) | H (%) | N (%) |
|---|---|---|---|
| Calculated for $C_{18}H_{24}N_4O_{10}\cdot 1.0H_2O$ | 45.57 | 5.52 | 11.81 |
| Found | 45.82 | 5.40 | 11.43 |

Example 9

Following a procedure similar to that of Example 3 but using 0.107 g of 4-(2-acetamido-3,4,6-tri-O-acetyl-2-deoxy-β-D-glucopyranosyl)oxy-1H-imidazole-5-carboxamide there was obtained 0.076 g of 4-(2-acetamido-2-deoxy-β-D-glucopyranosyl)oxy-1H-imidazole-5-carboxamide. m.p. 140°C. (dec.)

$\nu_{max}^{nujol}$ ($cm^{-1}$): 3440, 1640, 1600, 1500, 1330, 1110, 1065, 1010

Example 10

Following a procedure similar to that of Example 1 but using 2.88 g of 5-carbamoyl-1-(4-nitrobenzyl)imidazolium-4-olate and 7.77 g of 2,3,4-tri-O-acetyl-$\alpha$-L-rhamnopyranosyl bromide there was obtained 3.32 g of of 1-(4-nitrobenzyl)-4-(2,3,4-tri-O-acetyl-$\alpha$-L-rhamnopyranosyl)oxy-1H-imidazole-5-carboxamide.

Recrystallization from chloroform—n-hexane. m.p. 103—105°C.

$\nu_{max}^{nujol}$ ($cm^{-1}$): 3475, 1750, 1660, 1600, 1520, 1440, 1240, 1220, 1050, 960

| Elemental analysis: | C (%) | H (%) | N (%) |
|---|---|---|---|
| Calculated for $C_{23}H_{26}N_4O_{11} \cdot 0.3H_2O$ | 51.17 | 4.97 | 10.38 |
| Found | 51.59 | 5.07 | 10.03 |

Example 11

Following a procedure similar to that of Example 2 but using 1.728 g of 1-(4-nitrobenzyl)-4-(2,3,4-tri-O-acetyl-$\alpha$-L-rhamnopyranosyl)oxy-1H-imidazole-5-carboxamide there was obtained 1.258 g of 4-(2,3,4-tri-O-acetyl-$\alpha$-L-rhamnopyranosyl)oxy-1H-imidazole-5-carboxamide.

Recrystallization from chloroform—n-hexane. m.p. 183.5—185.5°C.

$\nu_{max}^{nujol}$ ($cm^{-1}$): 3460, 3170, 1750, 1650, 1600, 1240, 1220

| Elemental analysis: | C (%) | H (%) | N (%) |
|---|---|---|---|
| Calculated for $C_{16}H_{21}N_3O_9 \cdot 0.2H_2O$ | 47.69 | 5.35 | 10.43 |
| Found | 47.57 | 5.38 | 10.30 |

Example 12

Following a procedure similar to that of Example 3 but using 1.03 g of 4-(2,3,4-tri-O-acetyl-$\alpha$-L-rhamnopyranosyl)oxy-1H-imidazole-5-carboxamide there was obtained 0.623 g of 4-$\alpha$-L-rhamnopyranosyloxy-1H-imidazole-5-carboxamide. m.p. 115°C. (dec.)

$\nu_{max}^{nujol}$ ($cm^{-1}$): 3460, 3400—3050, 1650, 1600, 1500, 1330, 1140, 1115, 1060, 960

| Elemental analysis: | C (%) | H (%) | N (%) |
|---|---|---|---|
| Calculated for $C_{10}H_{15}N_3O_6 \cdot 5H_2O$ | 42.55 | 5.71 | 14.89 |
| Found | 42.77 | 5.78 | 14.87 |

Example 13

Following a procedure similar to that of Example 1 but using 1.017 g of 4-carbamoylimidazolium-5-olate and 2,3,5-tri-O-benzoyl-D-arabinofuranosyl bromide there was obtained 0.9 g of 4-(2,3,5-tri-O-benzoyl-$\alpha$-D-arabinofuranosyl)oxy-1H-imidazole-5-carboxamide.

Recrystallization from chloroform—ether. m.p. 215—216°C.

$\nu_{max}^{nujol}$ ($cm^{-1}$): 3460, 3360, 1720, 1665, 1610, 1280, 1120, 980, 705

| Elemental analysis: | C (%) | H (%) | N (%) |
|---|---|---|---|
| Calculated for $C_{30}H_{25}N_3O_9$ | 63.05 | 4.41 | 7.35 |
| Found | 62.74 | 4.43 | 7.22 |

Example 14

Following a procedure similar to that of Example 3 but using 0.114 g of 4-(2,3,5-tri-O-benzoyl-$\alpha$-D-arabinofuranosyl)oxy-1H-imidazole-5-carboxamide there was obtained 0.045 g of 4-$\alpha$-D-arabinofuranosyloxy-1H-imidazole-5-carboxamide. m.p. 80°C.

$\nu_{max}^{nujol}$ ($cm^{-1}$): 3450—3150, 1650, 1600, 1110, 1070, 960

| Elemental analysis: | C (%) | H (%) | N (%) |
|---|---|---|---|
| Calculated for $C_9H_{13}N_3O_6 \cdot 1.1H_2O$ | 38.74 | 5.49 | 15.06 |
| Found | 38.65 | 5.13 | 14.78 |

Example 15

Following a procedure similar to that of Example 1 but using 4-carbamoyl-imidazolium-5-olate and 2,3,4,6-tetra-O-acetyl-$\alpha$-D-glucopyranosyl bromide there was obtained 4-(2,3,4,6-tetra-O-acetyl-$\beta$-D-glucopyranosyl)oxy-1H-imidazole-5-carboxamide. This product was identified to be same as that obtained in Example 2.

Example 16

Following a procedure similar to that of Example 1 but using 4-carbamoyl-imidazolium-5-olate and 2,3,4,6-tetra-O-acetyl-$\alpha$-D-mannopyranosyl chloride there was obtained 4-(2,3,4,6-tetra-O-acetyl-$\alpha$-D-mannopyranosyl)oxy-1H-imidazole-5-carboxamide. m.p. 148—154°C. (92°C. sintering)

$\nu_{max}^{nujol}$ ($cm^{-1}$): 3460, 3150, 1750, 1645, 1595

| Elemental analysis: | C (%) | H (%) | N (%) |
|---|---|---|---|
| Calculated for $C_{18}H_{23}N_3O_{11} \cdot 2H_2O$ | 43.81 | 5.51 | 8.52 |
| Found | 43.45 | 4.97 | 7.94 |

Example 17

Following a procedure similar to that of Example 1 but using 4-carbamoyl-imidazolium-5-olate and 3,4,6-tri-O-acetyl-2-deoxy-2-phthalimido-$\beta$-D-glucopyranosyl chloride there was obtained 4-(3,4,6-tri-O-acetyl-2-deoxy-2-phthalimido-$\beta$-D-glucopyranosyl)oxy-1H-imidazole-5-carboxamide. m.p. 202—208°C.

$\nu_{max}^{nujol}$ ($cm^{-1}$): 3460, 3270, 1780, 1750, 1735, 1710, 1665, 1600

| Elemental analysis: | C (%) | H (%) | N (%) |
|---|---|---|---|
| Calculated for $C_{24}H_{24}N_4O_{10} \cdot 2H_2O$ | 51.06 | 5.00 | 9.93 |
| Found | 51.23 | 4.48 | 9.72 |

Example 18

Following a procedure similar to that of Example 1 but using 1.442 g of 5-carbamoyl-1-(4-nitrobenzyl)imidazolium-4-olate and 4.37 g of methyl 1-bromo-1-deoxy-2,3,4-tri-O-acetyl-$\alpha$-D-glucopyranuronate there was obtained methyl 1-O-[5-carbamoyl-1-(4-nitrobenzyl)imidazole-4-yl]-2,3,4-tri-O-acetyl-$\beta$-D-glucopyranuronate. The resulting product as such was used as the starting compound in the following Example 19.

Example 19

Following a procedure similar to that of Example 2 but using 1.157 g of methyl 1-O-[5-carbamoyl-1-(4-nitrobenzyl)imidazole-4-yl]-2,3,4-tri-O-acetyl-$\beta$-D-glucopyranuronate there was obtained 0.825 g of methyl 1-O-(5-carbamoyl-1H-imidazole-4-yl)-2,3,4-tri-O-acetyl-$\beta$-D-glucopyranuronate. m.p. 211—212°C.

$\nu_{max}^{nujol}$ ($cm^{-1}$): 3450, 3350, 3175, 1760, 1655, 1605, 1500, 1440, 1220, 1100, 1040

Example 20

Following a procedure similar to that of Example 3 but using 0.85 g of methyl 1-O-(5-carbamoyl-1H-imidazole-4-yl)-2,3,4-tri-O-acetyl-$\beta$-D-glucopyranuronate there was obtained 0.55 g of methyl 1-O-(5-carbamoyl-1H-imidazole-4-yl)-$\beta$-D-glucopyranuronate. m.p. 146°C. (dec.)

$\nu_{max}^{nujol}$ ($cm^{-1}$): 3450, 3330, 1740, 1650, 1600, 1085, 1020

| Elemental analysis: | C (%) | H (%) | N (%) |
|---|---|---|---|
| Calculated for $C_{11}H_{15}N_3O_8 \cdot \frac{3}{4}H_2O$ | 39.94 | 5.03 | 12.70 |
| Found | 40.12 | 4.80 | 12.13 |

Example 21

A solution of 0.55 g of methyl 1-O-(5-carbamoyl-1H-imidazole-4-yl)-β-D-glucopyranuronate in 50 ml of dry methanol was saturated with ammonia (gas) with cooling in an ice bath and the reaction mixture was stirred for 1.5 hours. Then the reaction mixture was heated to room temperature and was concentrated to a residue under reduced pressure. The residue was washed with methanol and ether, and then separated crystals were filtered off and dried to give 0.5 g of 1-O-(5-carbamoyl-1H-imidazole-4-yl)-β-D-glucopyranuronamide which was recrystallized from water—ethanol. m.p. 206°C. (dec.)

$\nu_{max}^{nujol}$ ($cm^{-1}$): 3590, 3410, 3170, 1690, 1660, 1610, 1340, 1155, 1070, 1040, 1025, 1010, 800, 790

| Elemental analysis: | C (%) | H (%) | N (%) |
|---|---|---|---|
| Calculated for $C_{10}H_{14}N_4O_7 \cdot 1H_2O$ | 37.50 | 5.04 | 17.49 |
| Found | 37.43 | 5.14 | 17.55 |

Example 22

A solution of 0.635 g of methyl 1-O-(5-carbamoyl-1H-imidazole-4-yl)-β-D-glucopyranuronate in 20 ml of dry methanol was saturated with methylamine (gas) with cooling in an ice bath and the reaction mixture was stirred for 2 hours. Separated crystals filtered off and dried to give 0.6 g of N-methyl [1-O-(5-carbamoyl-1H-imidazole-4-yl)-β-D-glucopyran]uronamide which was recrystallized from water—n-propanol. m.p. 168°C. (dec.)

$\nu_{max}^{nujol}$ ($cm^{-1}$): 3490, 3450, 3350, 3250, 1660, 1640, 1610, 1515, 1440, 1420, 1170, 1130, 1110, 1085, 1070, 1020, 770

| Elemental analysis: | C (%) | H (%) | N (%) |
|---|---|---|---|
| Calculated for $C_{11}H_{16}N_4O_7 \cdot 1H_2O$ | 39.52 | 5.43 | 16.76 |
| Found | 39.28 | 5.41 | 16.55 |

Example 23

A solution of 0.635 g of methyl 1-O-(5-carbamoyl-1H-imidazole-4-yl)-β-D-glucopyranuronate in 12 ml of methanol, 7.4 ml of water and 0.6 ml of 28%-ammonia water was stirred for 24 hours at a room temperature. The reaction mixture was concentrated to a residue under reduced pressure below 40°C. and the residue was crystallized with methanol giving 0.5 g of ammonium 1-O-(5-carbamoyl-1H-imidazole-4-yl)-β-D-glucopyranuronate which was recrystallized from water—methanol. m.p. 185°C. (dec.)

$\nu_{max}^{nujol}$ ($cm^{-1}$): 3440, 3400, 3330, 3280, 3220, 1660, 1620, 1580, 1515, 1400, 1100, 1070, 1040, 1020

| Elemental analysis: | C (%) | H (%) | N (%) |
|---|---|---|---|
| Calculated for $C_{10}H_{16}N_4O_8$ | 37.50 | 5.04 | 17.49 |
| Found | 37.69 | 5.27 | 16.96 |

Example 24

Following a procedure similar to that of Example 1 but using 2,3,5-tri-O-acetyl-D-ribofuranosyl-chloride prepared from 2.50 g of 1,2,3,5-tetra-O-acetyl-β-D-ribofuranose and 1.022 g of 5-carbamoyl-1-(4-nitrobenzyl)imidazolium-4-olate there was obtained 0.986 g of 1-(4-nitrobenzyl)-4-(2,3,5-tri-O-acetyl-β-D-ribofuranosyl)oxy-1H-imidazole-5-carboxamide which was recrystallized from ethanol. m.p. 155.5—156.5°C.

| Elemental analysis: | C (%) | H (%) | N (%) |
|---|---|---|---|
| Calculated for $C_{22}H_{24}N_4O_{11} \cdot \frac{1}{2}H_2O$ | 49.90 | 4.76 | 10.58 |
| Found | 50.09 | 4.63 | 10.60 |

Example 25

Following a procedure similar to that of Example 2 but using 3.360 g of 1-(4-nitrobenzyl)-4-(2,3,5-tri-O-acetyl-$\beta$-D-ribofuranosyl)oxy-1H-imidazole-5-carboxamide there was obtained 1.829 g of 4-(2,3,5-tri-O-acetyl-$\beta$-D-ribofuranosyl)oxy-1H-imidazole-5-carboxamide which was recrystallized from chloroform—isopropyl ether. m.p. 132—133.5°C.

$\nu_{max}^{nujol}$ ($cm^{-1}$): 3470, 3320, 3150, 1745, 1650, 1600, 1570

| Elemental analysis: | C (%) | H (%) | N (%) |
|---|---|---|---|
| Calculated for $C_{15}H_{19}N_3O_9$ | 46.75 | 4.97 | 10.91 |
| Found | 46.54 | 5.08 | 10.58 |

$\lambda_{max}$ in water 250 nm ($\varepsilon$ 13400)
$\lambda_{max}$ in N—HCl aqueous solution 241 nm ($\varepsilon$ 9700)
$\lambda_{max}$ in N—NaOH aqueous solution 265 nm ($\varepsilon$ 13300)

Example 26

Following a procedure similar to that of Example 3 but using 1.156 g of 4-(2,3,5-tri-O-acetyl-$\beta$-D-ribofuranosyl)oxy-1H-imidazole-5-carboxamide there was obtained 0.734 g of 4-$\beta$-D-ribofuranosyloxy-1H-imidazole-5-carboxamide. m.p. 167°C. (dec.)

$\nu_{max}^{KBr}$ ($cm^{-1}$): 3450, 3350, 3260, 1670, 1595, 1505

| Elemental analysis: | C (%) | H (%) | N (%) |
|---|---|---|---|
| Calculated for $C_9H_{13}N_3O_6 \cdot 3/5H_2O$ | 40.03 | 5.30 | 15.56 |
| Found | 39.62 | 4.90 | 15.35 |

$\lambda_{max}$ in water 251 nm ($\varepsilon$ 11800)
$\lambda_{max}$ in N—HCl aqueous solution 242 nm ($\varepsilon$ 8100)
$\lambda_{max}$ in N—NaOH aqueous solution 265 nm ($\varepsilon$ 11600)

According to the present invention, there are similarly obtainable, for example, the compounds:
4-(2,3,5-tri-O-benzoyl-$\beta$-D-ribofuranosyl)oxy-1H-imidazole-5-carboxamide;
4-$\alpha$-D-mannopyranosyloxy-1H-imidazole-5-carboxamide;
1-O-(5-carbamoyl-1H-imidazole-4-yl)-$\beta$-D-glucopyranuronic acid;
4-(2-deoxy-2-phthalimido-$\beta$-D-glucopyranosyl)oxy-1H-imidazole-5-carboxamide;
4-(2,3,4-tri-O-acetyl-$\beta$-D-ribopyranosyl)oxy-1H-imidazole-5-carboxamide;
4-$\beta$-D-ribopyranosyloxy-1H-imidazole-5-carboxamide;
4-(2,3,5-tri-O-acetyl-$\beta$-D-xylofuranosyl)oxy-1H-imidazole-5-carboxamide;
4-$\beta$-D-xylofuranosyloxy-1H-imidazole-5-carboxamide;
4-(2,3,4-tri-O-acetyl-$\beta$-D-xylopyranosyl)oxy-1H-imidazole-5-carboxamide;
4-$\beta$-D-xylopyranosyl-1H-imidazole-5-carboxamide;
4-(2,3,4-tri-O-acetyl-$\alpha$-D-arabinopyranosyl)oxy-1H-imidazole-5-carboxamide;
4-$\alpha$-D-arabinopyranosyloxy-1H-imidazole-5-carboxamide;
4-(2,3,5-tri-O-acetyl-$\alpha$-D-lyxofuranosyl)oxy-1H-imidazole-5-carboxamide;
4-$\alpha$-D-lyxofuranosyloxy-1H-imidazole-5-carboxamide;
4-(2,3,4-tri-O-acetyl-$\alpha$-D-lyxopyranosyl)oxy-1H-imidazole-5-carboxamide;
4-$\alpha$-D-lyxopyranosyloxy-1H-imidazole-5-carboxamide;
4-(2-deoxy-3,5-di-O-p-nitrobenzoyl-$\alpha$-D-ribofuranosyl)oxy-1H-imidazole-5-carboxamide;
4-(2-deoxy-3,5-di-O-p-nitrobenzoyl-$\beta$-D-ribofuranosyl)oxy-1H-imidazole-5-carboxamide;
4-(2-deoxy-$\alpha$-D-ribofuranosyl)oxy-1H-imidazole-5-carboxamide;
4-(2-deoxy-$\beta$-D-ribofuranosyl)oxy-1H-imidazole-5-carboxamide;
4-(2,3-di-O-acetyl-5-deoxy-$\beta$-D-ribofuranosyl)oxy-1H-imidazole-5-carboxamide; and
4-(5-deoxy-$\beta$-D-ribofuranosyl)oxy-1H-imidazole-5-carboxamide;

**Claims**

1. A compound of the formula

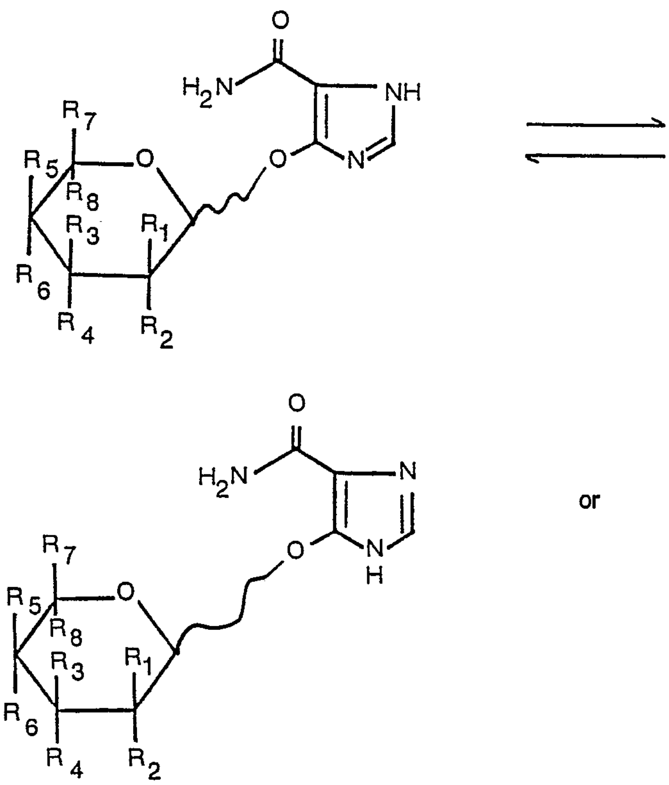

or

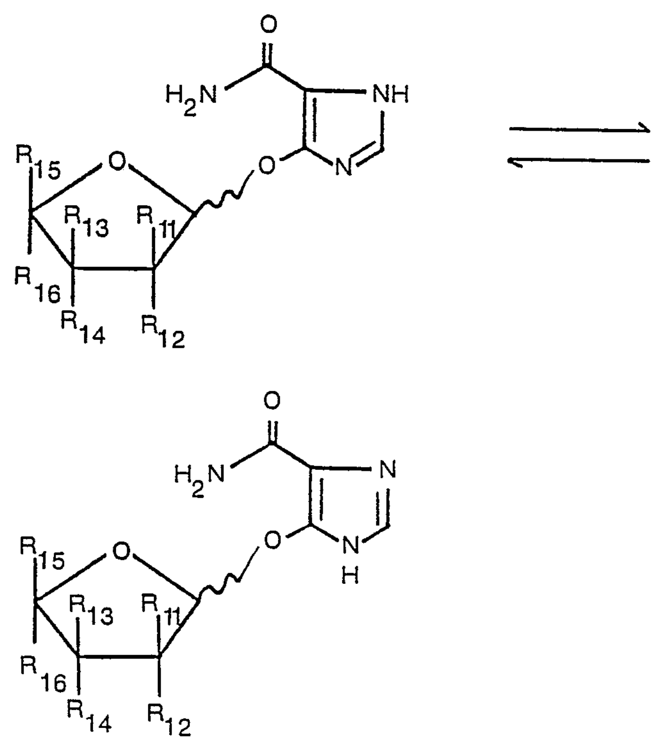

wherein either $R_1$ or $R_2$ is a hydrogen atom, a hydroxy group, an acyloxy group, a phthalimido group or an acetamido group and the other is a hydrogen atom; either $R_3$ or $R_4$ is a hydroxy group or an acyloxy group and the other is a hydrogen atom; either $R_5$ or $R_6$ is a hydroxy group or an acyloxy group and the other is a hydrogen atom; either $R_7$ or $R_8$ is a hydrogen atom, a methyl group, a hydroxymethyl group, an acyloxymethyl group, an alkoxycarbonyl group, a group of the formula:

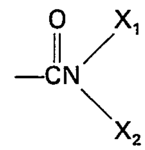

wherein $X_1$ and $X_2$ are the same or different and selected from a hydrogen atom and $C_1$ to $C_6$ alkyl groups, or a carboxyl group and the other is a hydrogen atom; either $R_{11}$ or $R_{12}$ is a hydrogen atom, a hydroxy group or an acyloxy group and the other is a hydrogen atom; either $R_{13}$ or $R_{14}$ is a hydroxy group or an acyloxy group and the other is a hydrogen atom; and either $R_{15}$ or $R_{16}$ is a methyl group, a hydroxymethyl group or an acyloxymethyl group and the other is a hydrogen atom.

2. At least one of the following compounds according to claim 1:
4-(2,3,4,6-tetra-O-acetyl-β-D-glucopyranosyl)oxy-1H-imidazole-5-carboxamide;
4-β-D-glucopyranosyloxy-1H-imidazole-5-carboxamide;
4-(2,3,4,6-tetra-O-acetyl-β-D-galactopyranosyl)oxy-1H-imidazole-5-carboxamide;
4-β-D-galactopyranosyloxy-1H-imidazole-5-carboxamide;
4-(2-acetamido-3,4,6-tri-O-acetyl-2-deoxy-β-D-glucopyranosyl)oxy-1H-imidazole-5-carboxamide;
4-(2-acetamido-2-deoxy-β-D-glucopyranosyl)oxy-1H-imidazole-5-carboxamide;
4-(2,3,4-tri-O-acetyl-α-L-rhamnopyranosyl)oxy-1H-imidazole-5-carboxamide;
4-α-L-rhamnopyranosyloxy-1H-imidazole-5-carboxamide;
4-(2,3,4,6-tetra-O-acetyl-α-D-mannopyranosyl)oxy-1H-imidazole-5-carboxamide;
4-(3,4,6-tri-O-acetyl-2-deoxy-2-phthalimido-β-D-glucopyranosyl)oxy-1H-imidazole-5-carboxamide;
Methyl 1-O-(5-carbamoyl-1H-imidazole-4-yl)-2,3,4-tri-O-acetyl-β-D-glucopyranuronate;
Methyl 1-O-(5-carbamoyl-1H-imidazole-4-yl)-β-D-glucopyranuronate;
1-O-(5-carbamoyl-1H-imdazole-4-yl)-β-D-glucopyranuronamide;
N-methyl[1-O-(5-carbamoyl-1H-imidazole-4-yl)-β-D-glucopyran]uronamide;
Ammonium 1-O-(5-carbamoyl-1H-imidazole-4-yl)-β-D-glucopyranuronate;
4-(2,3,5-tri-O-benzoyl-α-D-arabinofuranosyl)oxy-1H-imidazole-5-carboxamide;
4-α-D-arabinofuranosyloxy-1H-imidazole-5-carboxamide
4-(2,3,5-tri-O-acetyl-β-D-ribofuranosyl)oxy-1H-imidazole-5-carboxamide, and
4-β-D-ribofuranosyloxy-1H-imidazole-5-carboxamide.

3. A process for producing a compound according to claim 1 which comprises reacting a 4-carbamoylimidazolium-5-olate derivative of the formula:

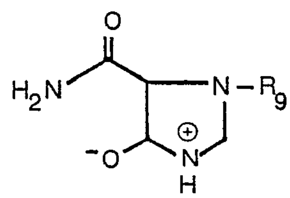

wherein $R_9$ is a hydrogen atom or a substituted or unsubstituted benzyl group; or a reactive derivative thereof with a carbohydrate derivative of formula:

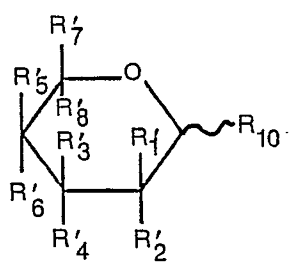

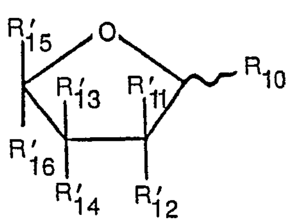

wherein either $R'_1$ or $R'_2$ is a hydrogen atom, an acyloxy group, a phthalimido group or an acetamido group and the other is a hydrogen atom; either $R'_3$ or $R'_4$ is an acyloxy group and the other is a hydrogen atom; either $R'_5$ or $R'_6$ is an acyloxy group and the other is a hydrogen atom; either $R'_7$ or $R'_8$ is a hydrogen atom, a methyl group, an acyloxymethyl group, an alkoxycarbonyl group or a group of the formula:

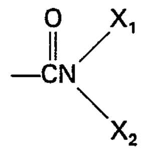

wherein $X_1$ and $X_2$ are as defined in claim 1, and the other is a hydrogen atom; either $R'_{11}$ or $R'_{12}$ is a hydrogen atom or an acyloxy group and the other is a hydrogen atom; either $R'_{13}$ or $R'_{14}$ is an acyloxy group and the other is a hydrogen atom; either $R'_{15}$ or $R'_{16}$ is a methyl group or an acyloxymethyl group and the other is a hydrogen atom; and $R_{10}$ is a halogen atom, a $C_1$ to $C_4$ alkoxy group or a $C_1$ to $C_7$ alkanoyloxy group to give a compound of formula:

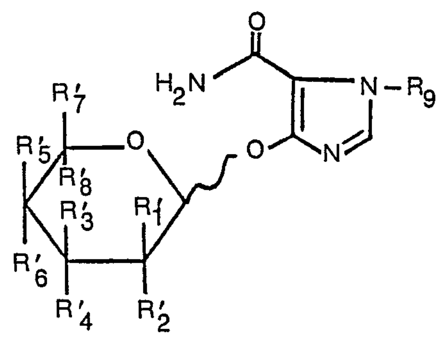

or

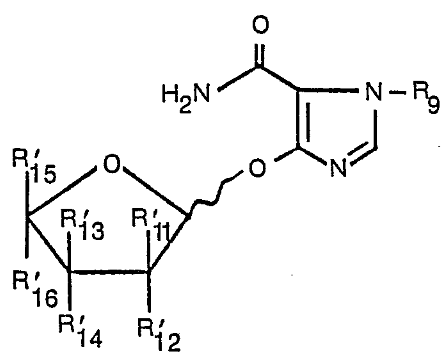

wherein $R'_1$, $R'_2$, $R'_3$, $R'_4$, $R'_5$, $R'_6$, $R'_7$, $R'_8$, $R'_{11}$, $R'_{12}$, $R'_{13}$, $R'_{14}$, $R'_{15}$, $R'_{16}$, $R_9$ are as defined above; optionally removing the benzyl protective group by catalytic reduction when $R_9$ is a substituted or unsubstituted benzyl group, optionally removing any protective group in the sugar moiety by deacylation under basic conditions, and optionally forming a carboxyl or amide derivative when either $R'_7$ or $R'_8$ is an alkoxycarbonyl group.

4. An antitumor composition which comprises an antitumor effective amount of a compound according to claim 1 or 2 as an active ingredient and pharmaceutically acceptable carrier or diluent.

5. An immunosuppressant composition which comprises an immunosuppressive amount of a compound according to claim 1 or 2 as an active ingredient and pharmaceutically acceptable carrier or diluent.

6. An antiviral composition which comprises an effective amount of a compound according to claim 1 or 2 as an active ingredient and pharmaceutically acceptable carrier or diluent.

**Revendications**

1. Composés de formule

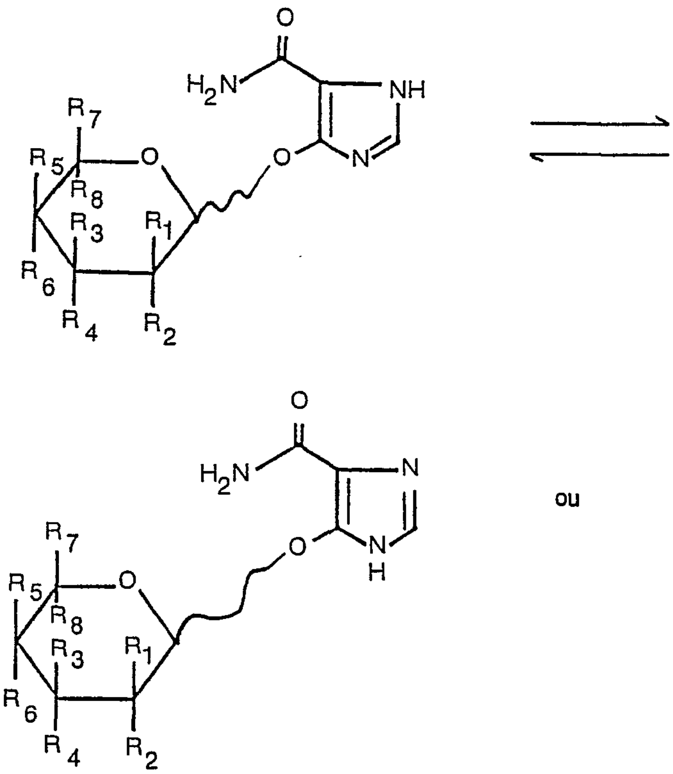

ou

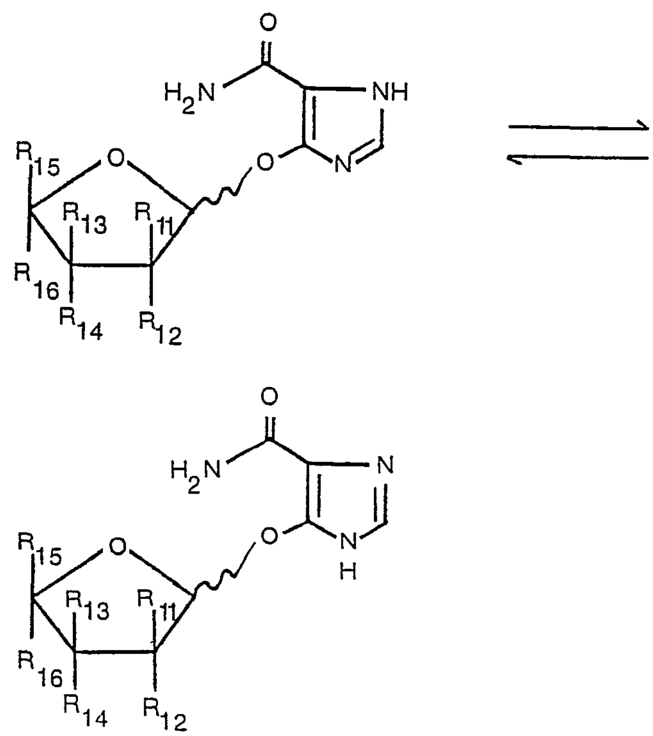

dans laquelle l'un de $R_1$ et $R_2$ est un atome d'hydrogène, un groupe hydroxy, un groupe acyloxy, un groupe phtalimido ou un groupe acétamido et l'autre est un atome d'hydrogène; l'un de $R_3$ et $R_4$ est un groupe hydroxy ou un groupe acyloxy et l'autre est un atome d'hydrogène; l'un de $R_5$ et $R_6$ est un groupe hydroxy ou un groupe acyloxy et l'autre est un atome d'hydrogène; l'un de $R_7$ et $R_8$ est un atome

d'hydrogène, un groupe méthyle, un groupe hydroxyméthyle, un groupe acyloxyméthyle, un groupe alkoxycarbonyle, un groupe de formule:

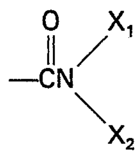

dans laquelle $X_1$ et $X_2$ sont égaux ou différents et sont choisis entre un atome d'hydrogène et des groupes alkyle en $C_1$ à $C_6$, ou un groupe carboxyle, et l'autre est un atome d'ydrogène; l'un de $R_{11}$ et $R_{12}$ est un atome d'hydrogène, un groupe hydroxy ou un groupe acyloxy et l'autre est un atome d'hydrogène; l'un de $R_{13}$ et $R_{14}$ est un groupe hydroxy ou un groupe acyloxy et l'autre est un atome d'hydrogène; et l'un de $R_{15}$ et $R_{16}$ est un groupe méthyle, un groupe hydroxyméthyle ou un groupe acyloxyméthyle et l'autre est un atome d'hydrogène.

2. L'un au moins des composés suivants, conformément à la revendication 1:
4-(2,3,4,6-tétra-O-acétyl-β-D-glucopyrannosyl)oxy-1H-imidazole-5-carboxamide;
4-β-D-glucopyrannosyloxy-1H-imidazole-5-carboxamide;
4-(2,3,4,6-tétra-O-acétyl-β-D-galactopyrannosyl)oxy-1H-imidazole-5-carboxamide;
4-β-D-galactopyrannosyloxy-1H-imidazole-5-carboxamide;
4-(2-acétamido-3,4,6-tri-O-acétyl-2-déoxy-β-D-glycopyrannosyl)oxy-1H-imidazole-5-carboxamide;
4-(2-acétamido-2-déoxy-β-D-glucopyrannosyl)oxy-1H-imidazole-5-carboxamide;
4-(2,3,4-tri-O-acétyl-α-L-rhamnopyrannosyl)oxy-1H-imidazole-5-carboxamide;
4-α-L-rhamnopyrannosyloxy-1H-imidazole-5-carboxamide;
4-(2,3,4,6-tétra-O-acétyl-α-D-mannopyrannosyl)oxy-1H-imidazole-5-carboxamide;
4-(3,4,6-tri-O-acétyl-2-déoxy-2-phtalimido-β-D-glucopyrannosyl)oxy-1H-imidazole-5-carboxamide;
1-O-(5-carbamoyl-1H-imidazole-4-yl)-2,3,4-tri-O-acétyl-β-D-glucopyrannuronate de méthyle;
1-O-(5-carbamoyl-1H-imidazole-4-yl)-β-D-glucopyrannuronate de méthyle;
1-O-(5-carbamoyl-1H-imidazole-4-yl)-β-D-glucopyrannuronamide;
N-méthyl[1-O-(5-carbamoyl-1H-imidazole-4-yl)-β-D-glucopyranne]uronamide;
1-O-(5-carbamoyl-1H-imidazole-4-yl)-β-D-glucopyrannuronate d'ammonium;
4-(2,3,5-tri-O-benzoyl-α-D-arabinofurannosyl)oxy-1H-imidazole-5-carboxamide;
4-α-D-arabinofurannosyloxy-1H-imidazole-5-carboxamide;
4-(2,3,5-tri-O-acétyl-β-D-ribofurannosyl)oxy-1H-imidazole-5-carboxamide; et
4-β-D-ribofurannosyloxy-1H-imidazole-5-carboxamide;

3. Procédé de production d'un composé suivant la revendication 1, qui consiste à faire réagir un 4-carbamoylimidazolium-5-olate de formule:

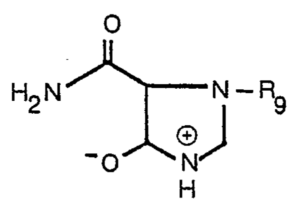

dans laquelle $R_9$ est un atome d'hydrogène ou un groupe benzyle substitué ou non substitué; ou l'un de ses dérivés réactifs avec un dérivé glucidique de formule:

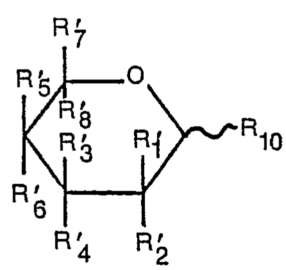

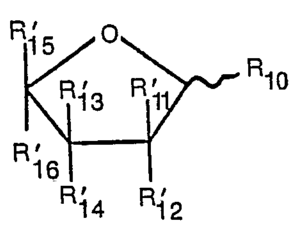

dans laquelle l'un de $R'_1$ et $R'_2$ est un atome d'hydrogène, un groupe acyloxy, un groupe phtalimido ou un groupe acétamido et l'autre est un atome d'hydrogène; l'un de $R'_3$ et $R'_4$ est un groupe acyloxy et l'autre est un atome d'hydrogène; l'un de $R'_5$ et $R'_6$ est un groupe acyloxy et l'autre est un atome d'hydrogène; l'un de $R'_7$ et $R'_8$ est un atome d'hydrogène, un groupe méthyle, un groupe acyloxyméthyle, un groupe alkoxycarbonyle ou un groupe de formule:

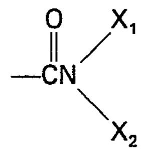

dans laquelle $X_1$ et $X_2$ ont les définitions données dans la revendication 1, et l'autre est un atome d'hydrogène; l'un de $R'_{11}$ et $R'_{12}$ est un atome d'hydrogène ou un groupe acyloxy et l'autre est un atome d'hydrogène; l'un de $R'_{13}$ et $R'_{14}$ est un groupe acyloxy et l'autre est un atome d'hydrogène; l'un de $R'_{15}$ et $R'_{16}$ est un groupe méthyle ou un groupe acyloxyméthyle et l'autre est un atome d'hydrogène; et $R_{10}$ est un atome d'halogène, un groupe alkoxy en $C_1$ à $C_4$ ou un groupe alcanoyloxy en $C_2$ à $C_7$, pour former un composé de formule:

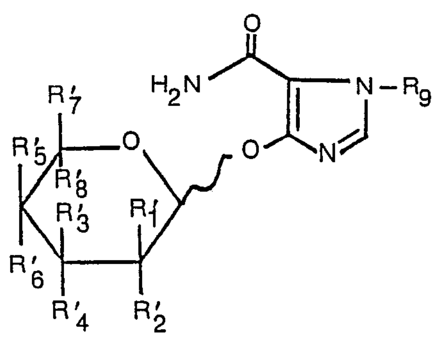

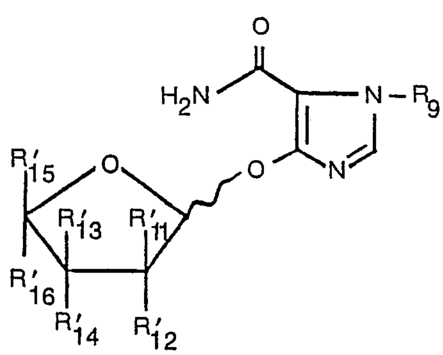

dans laquelle $R'_1$, $R'_2$, $R'_3$, $R'_4$, $R'_5$, $R'_6$, $R'_7$, $R'_8$, $R'_{11}$, $R'_{12}$, $R'_{13}$, $R'_{14}$, $R'_{15}$, $R'_{16}$, $R_9$ ont les définitions données ci-dessus; à éliminer éventuellement le groupe benzyle protecteur par réduction catalytique lorsque $R_9$ est un groupe benzyle substitué ou non substitué, à éliminer éventuellement tout groupe protecteur de la portion sucre par désacylation dans des conditions basiques, et à former éventuellement un dérivé carboxylique ou amidique lorsque l'un de $R'_7$ et $R'_8$ est un groupe alkoxycarbonyle.

4. Composition antitumorale, qui comprend une quantité à effet antitumorale d'un composé suivant la revendication 1 ou 2 comme ingrédient actif et un support ou diluant acceptable du point de vue pharmaceutique.

5. Composition immunosuppresive, qui comprend une quantité immunosuppressive d'un composé suivant la revendication 1 ou 2 comme ingrédient actif et un support ou diluant pharmaceutiquement acceptable.

6. Composition antivirale, qui comprend une quantité efficace d'un composé suivant la revendication 1 ou 2 comme ingédient actif et un support ou diluant pharmaceutiquement acceptable.

**Patentansprüche**

1. Verbindung der Formel

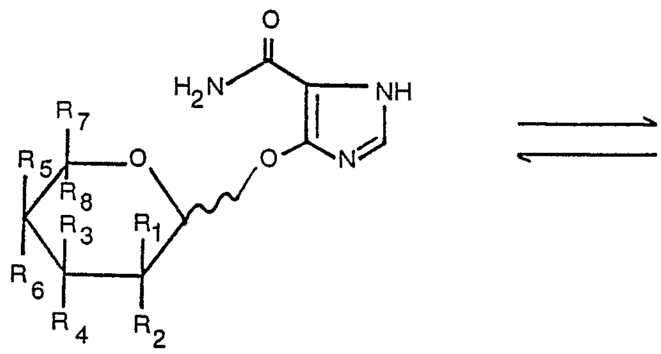

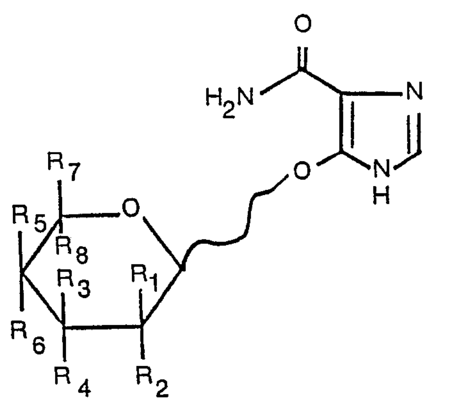

oder

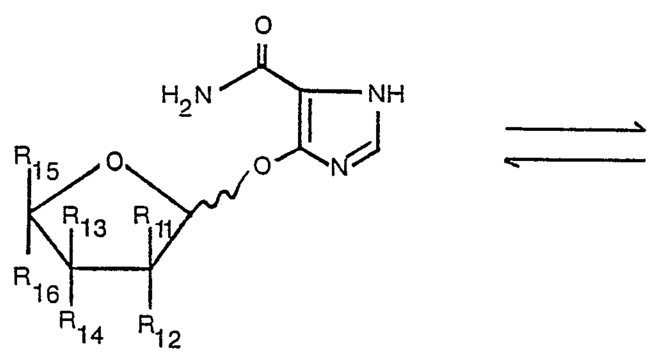

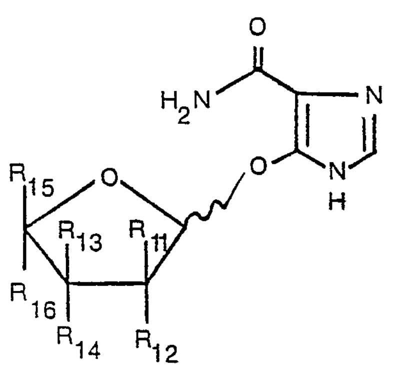

worin entweder $R_1$ oder $R_2$ für ein Wasserstoffatom, eine Hydroxygruppe, eine Acyloxygruppe, eine Phthalimidogruppe oder eine Acetamidogruppe stehen un der andere Substituiert ein Wasserstoffatom ist, entweder $R_3$ oder $R_4$ eine Hydroxygruppe oder eine Acyloxygruppe bedeuten und der andere Substituent ein Wasserstoffatom ist, entweder $R_5$ oder $R_6$ eine Hydroxygruppe oder eine Acyloxygruppe sind

und der andere Substituent ein Wasserstoffatom bedeutet, entweder $R_7$ oder $R_8$ ein Wasserstoffatom, eine Methylgruppe, eine Hydroxymethylgruppe, eine Acyloxymethylgruppe, eine Alkoxycarbonylgruppe, eine Gruppe der Formel

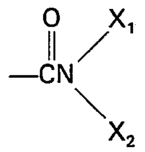

worin $X_1$ und $X_2$ gleich oder verschieden sind und aus einem Wasserstoffatom sowie $C_1$—$C_6$-Alkylgruppen ausgewählt sind, oder eine Carboxylgruppe ist und der andere Substituent ein Wasserstoffatom bedeutet, entweder $R_{11}$ oder $R_{12}$ ein Wasserstoffatom, eine Hydroxygruppe oder eine Acyloxygruppe sind und der andere Substituent ein Wasserstoffatom ist, entweder $R_{13}$ oder $R_{14}$ eine Hydroxygruppe oder eine Acyloxygruppe sind und der andere Substituent ein Wasserstoffatom ist, und entweder $R_{15}$ oder $R_{16}$ eine Methylgruppe, eine Hydroxymethylgruppe oder eine Acyloxymethylgruppe sind und der andere Substituent ein Wasserstoffatom ist.

2. Wenigstens eine der folgenden Verbindungen gemäß Anspruch 1:
4-(2,3,4,6-Tetra-O-acetyl-β-D-glucopyranosyl)oxy-1H-imidazol-5-carboxamid;
4-β-D-Glucopyranosyloxy-1H-imidazol-5-carboxamid;
4-(2,3,4,6-Tetra-O-acetyl-β-D-galactopyranosyl)oxy-1H-imidazol-5-carboxamid;
4-β-D-galactopyranosyloxy-1H-imidazol-5-carboxamid;
4-(2-Acetamido-3,4,6-tri-O-acetyl-2-deoxy-β-D-glucopyranosyl)oxy-1H-imidazol-5-carboxamid;
4-(2-Acetamido-2-deoxy-β-D-glucopyranosyl)oxy-1H-imidazol-5-carboxamid;
4-(2,3,4-Tri-O-acetyl-α-L-rhamnopyranosyl)oxy-1H-imidazol-5-carboxamid;
4-α-L-Rhamnopyranosyloxy-1H-imidazol-5-carboxamid;
4-(2,3,4,6-Tetra-O-acetyl-α-D-mannopyranosyl)oxy-1H-imidazol-5-carboxamid;
4-(3,4,6-Tri-O-acetyl-2-deoxy-2-phthalimido-β-D-glucopyranosyl)oxy-1H-imidazol-5-carboxamid;
Methyl-1-O-(5-carbamoyl-1H-imidazol-4-yl)-2,3,4-tri-O-acetyl-β-D-glucopyranuronat;
Methyl-1-O-(5-carbamoyl-1H-imidazol-4-yl)-β-D-glucopyranuronat;
1-O-(5-carbamoyl-1H-imidazol-4-yl)-β-D-glucopyranuronamid;
N-Methyl[1-O-(5-carbamoyl-1H-imidazol-4-yl)-β-D-glucopyran]uronamide;
Ammonium-1-O-(5-carbamoyl-1H-imidazol-4-yl)-β-D-glucopyranuronat;
4-(2,3,5-Tri-O-benzoyl-α-D-arabinofuranosyl)oxy-1H-imidazol-5-carboxamid;
4-α-D-Arabinofuranosyloxy-1H-imidazol-5-carboxamid;
4-(2,3,5-Tri-O-acetyl-β-D-ribofuranosyl)oxy-1H-imidazol-5-carboxamid und
4-β-D-ribofuranosyloxy-1H-imidazol-5-carboxamid.

3. Verfahren zur Herstellung einer Verbindung gemäß Anspruch 1, dadurch gekennzeichnet, daß ein 4-Carbamoylimidazolium-5-olatderivat der Formel

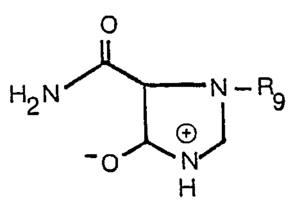

worin $R_9$ für ein Wasserstoffatom oder eine substituierte oder nichtsubstituierte Benzylgruppe steht, oder ein reaktives Derivat davon mit einem Kohlehydratderivat der Formel:

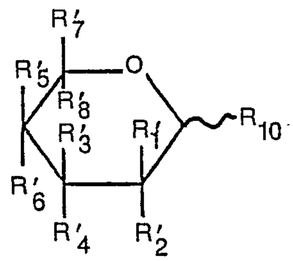

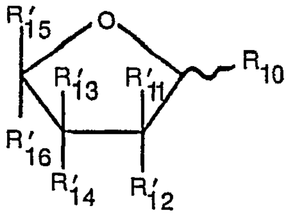

worin entweder $R'_1$ oder $R'_2$ ein Wasserstoffatom, eine Acyloxygruppe, eine Phthalimidogruppe oder eine Acetamidogruppe sind und der andere Substituent ein Wasserstoffatom bedeutet, entweder $R'_3$ oder $R'_4$ eine Acyloxygruppe bedeuten und der andere Substituent ein Wasserstoffatom ist, entweder $R'_5$ oder $R'_6$ eine Acylgruppe bedeuten und der andere Substituent ein Wasserstoffatom ist, entweder $R'_7$ oder $R'_8$ ein Wasserstoffatom, eine Methylgruppe, eine Acyloxymethylgruppe, eine Alkoxycarbonylgruppe oder eine Gruppe der Formel

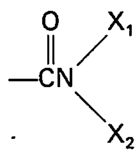

worin $X_1$ und $X_2$ die in Anspruch 1 angegebenen Bedeutungen besitzen, sind und der andere Substituent ein Wasserstoffatom ist, entweder $R'_{11}$ oder $R'_{12}$ ein Wasserstoffatom oder eine Acyloxygruppe sind und der andere Substituent ein Wasserstoffatom ist, entweder $R'_{13}$ oder $R'_{14}$ eine Acyloxygruppe sind und der andere Substituent ein Wasserstoffatom ist, entweder $R'_{15}$ oder $R'_{16}$ eine Methylgruppe oder eine Acyloxymethylgruppe sind und der andere Substituent ein Wasserstoffatom ist und $R_{10}$ ein Halogenatom, eine $C_1C_4$-Alkoxygruppe oder eine $C_2$—$C_7$-Alkanoyloxygruppe bedeuten, unter Gewinnung einer Verbindung der Formel:

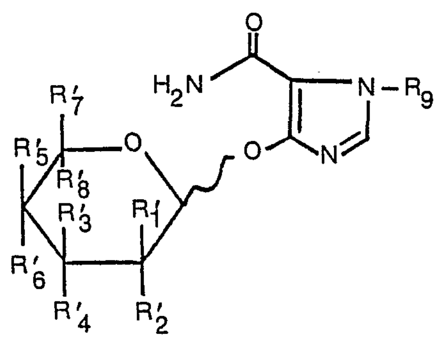

oder

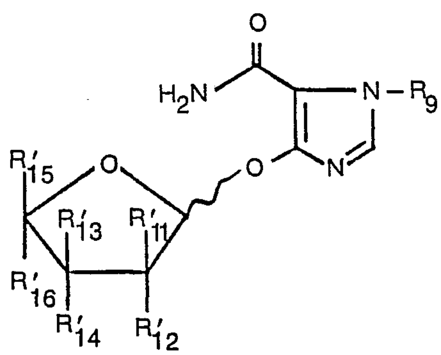

worin $R'_1$, $R'_2$, $R'_3$, $R'_4$, $R'_5$, $R'_6$, $R'_7$, $R'_8$, $R'_{11}$, $R'_{12}$, $R'_{13}$, $R'_{14}$, $R'_{15}$, $R'_{16}$, $R_9$ die vorstehend angegebenen Bedeutungen besitzen, umgesetzt wird, gegebenenfalls die schützende Benzylgruppe durch katalytische Reduktion entfernt wird, wenn $R_9$ eine substituierte oder nichtsubstituierte Benzylgruppe bedeutet, gegebenenfalls irgendeine schützende Gruppe in dem Zuckeranteil durch Deacylierung unter basischen Bedingungen entfernt wird, und gegebenenfalls ein Carboxyl- oder Amidderivat gebildet wird, wenn entweder $R'_7$ oder $R'_8$ eine Alkoxycarbonylgruppe bedeutet.

4. Antitumormittel aus einer Antitumor-wirksamen Menge einer Verbindung gemäß Anspruch 1 oder 2 als Wirkstoff und einem pharmazeutisch verträglichen Träger oder Verdünnungsmittel.

5. Immunosuppressantmittel aus einer immunosuppressiven Menge einer Verbindung gemäß Anspruch 1 oder 2 als Wirkstoff und einem pharmazeutisch verträglichen Träger oder Verdünnungsmittel.

6. Antivirusmittel aus einer wirksamen Menge einer Verbindung gemäß Anspruch 1 oder 2 als Wirkstoff und einem pharmazeutisch verträglichen Träger oder Verdünnungsmittel.